# EUROPEAN PATENT APPLICATION

(11) **EP 2 356 976 A2**
(43) Date of publication of application: **17.08.2011**
(21) Application number: 10186968.3
(22) Date of filing: 06.03.2009
(51) Int. Cl.: A61K 8/34, A61K 8/81, A61K 8/84, A61K 8/86, A61K 8/92, A61Q 9/02, A61K 8/36

(54) **Shaving aid material**

(30) Priority: 07.03.2008 US 34633 P
(62) Divisional of application: 09721179.1
(71) Applicant: Eveready Battery Company, Inc., St Louis, Missouri 63141 (US)
(72) Inventor: Coope-Epstein, Janet, Norwalk, CT 06851 (US); Jannusch, Leonard, White Bear Lake, MN 55110 (US)
(74) Representative: von Kreisler Selting Werner

(57) **Abstract**

A shaving aid material is provided that includes a water-soluble lubricious shaving aid in combination with a water-insoluble erodable medium. In some embodiments, the shaving aid material further includes a water-soluble thermoplastic polymer, and may also include a plasticizer. Optional additional ingredients include emulsifiers, surfactants, skin conditioners, fragrances, depilatory agents, cleaning agents, medicinal agents, etc.

## Description

### CROSS-REFERENCE TO RELATED APPLICATIONS

This application claims the benefit of U.S. Provisional Patent Application Serial No. 61/034,633, filed on March 7, 2008, the contents of which are incorporated by reference herein in their entirety.

### BACKGROUND OF THE INVENTION

### Technical Field

This invention relates to shaving devices in general, and to shaving aids used with shaving devices in a wet environment in particular.

### Background Information

Modern safety razors include a plurality of blades disposed within a cartridge that is pivotally or rigidly mounted on a handle. Some safety razors have a disposable cartridge for use with a reusable handle, while others have a handle and cartridge that are combined into a unitary disposable. Although a variety of razor cartridge configurations exist, most include a frame made of a rigid plastic that includes a seat and a cap. Cartridges often include a guard disposed forward of the blades. The guard and the cap orient the position of the person's skin relative to the blades to optimize the shaving action of the blade. Some cartridges include a shaving aid strip (sometimes referred to as a "comfort strip") comprised of shaving aids (e.g., lubricating agents, drag reducing agents, depilatory agents, cleaning agents, medicinal agents, etc.) to enhance the shaving process. Other shaving systems, such as the Intuition®, utilize a body of shaving aid material disposed entirely around a razor cartridge. The terms "forward" and "aft", as used herein, define relative position between features of the safety razor (i.e., razor assembly). A feature "forward" of the razor blades, for example, is positioned so that the surface to be shaved encounters the feature before it encounters the razor blades, if the razor assembly is being stroked in its intended cutting direction (e.g., the guard is forward of the razor blades). A feature "aft" of the razor blades is positioned so that the surface to be shaved encounters the feature after it encounters the razor blades, if the razor assembly is being stroked in its intended cutting direction (e.g., the cap is disposed aft of the razor blades).

The comfort and performance provided by a particular razor are critical to the commercial success of the razor. Improvements that benefit razor comfort, performance, and ease of use, however significant or subtle, can have a decided impact on the commercial success of a razor. For example, the usable life of a cartridge is often limited by the usable life of the shaving aid strip. Once the shaving aid strip is consumed, it is generally believed that the razor must also be in need of replacement. In fact, razor blades within currently available razor assemblies are very often still fit for use after the shaving aid strip has been consumed

It is known that shaving aid strips may be formed into a solid comprising at least one water-soluble polymeric component, such as polyethylene oxide, and a water-insoluble polymeric matrix comprising, for example, polystyrene. Upon exposure to water, the water-soluble shaving aid leaches from the composite onto the skin, thereby depositing the materials that facilitate the shaving process. This type of shaving aid strip works well until the water-soluble shaving aid material is substantially depleted, after which time the water-insoluble polymeric matrix is left to contact the user's skin with little or no assistance from the water-soluble materials. The result is a less than desirable shave experience.

In addition to providing a less than desirable usable life, shaving aid materials that include a relatively high molecular weight polymeric material as a matrix (e.g., polystyrene) also require processing temperatures that cause degradation of shaving aid materials such as polyethylene oxide and functional additives such as plasticizers, moisturizers, perfumes, and vitamins. Common processing temperatures for the extrusion of prior art shaving strips are often approximately 200°C. Efforts have been made to reduce the processing temperatures by manufacturing the shaving strips in, for example, a core-slleath configuration. For example, U.S. Patent No. 6,298,558 to Tseng, discloses extrusion temperatures as low as 130°C. However, degradation of the shaving aid materials at even these reduced temperatures still occurs.

What is needed, therefore, is a razor shaving aid material that will last for an extended period of time, one that will provide a desirable shaving experience, and one that can be manufactured at temperatures low enough to avoid appreciable thermal degradations of ingredients.

### DISCLOSURE OF THE INVENTION

It is, therefore, an object of the present invention to provide a razor assembly cartridge that provides a desirable perception of quality.

According to the present invention, a shaving aid material is provided that includes a water-soluble lubricious shaving aid in combination with a water-insoluble erodable medium. In some embodiments, the shaving aid material further includes a water-soluble thermoplastic polymer, and may also include a plasticizer. Optional additional ingredients include emulsifiers, surfactants, skin conditioners, fragrances, depilatory agents, cleaning agents, medicinal agents, etc., as will be described below.

### BRIEF DESCRIPTION OF THE DRAWINGS

FIG. 1 is a perspective view of a razor assembly.
FiG. 2 is a top view of a razor cartridge embodiment, including a shaving aid strip disposed in the cap, aft of the razor blades.
FIG. 3 is a top view of a razor cartridge embodiment, including a shaving aid strip disposed within the cartridge around the periphery of the razor blades.
FIG. 4 is a perspective view of the razor cartridge shown in FIG. 3 without the shaving aid embodiment disposed within the cartridge to clearly show the positioning of the shaving aid material.
FIG. 5 is a side view of a razor assembly having a shaving aid body disposed around a razor cartridge.
FIG. 6 is a partial perspective view of the razor cartridge and shaving aid body shown in FIG. 5.

### DETAILED DESCRIPTION OF THE INVENTION

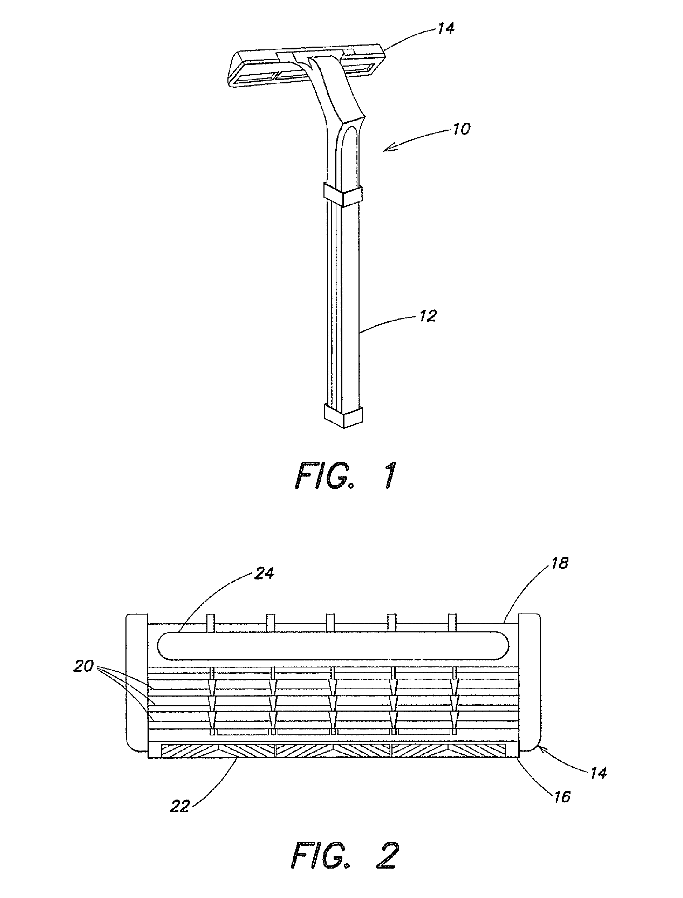

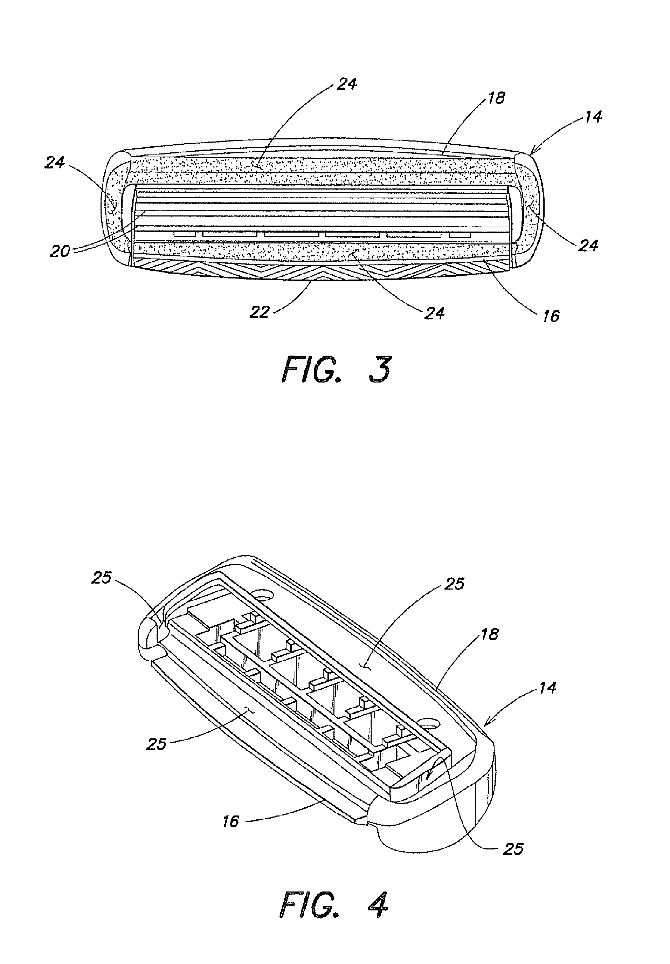

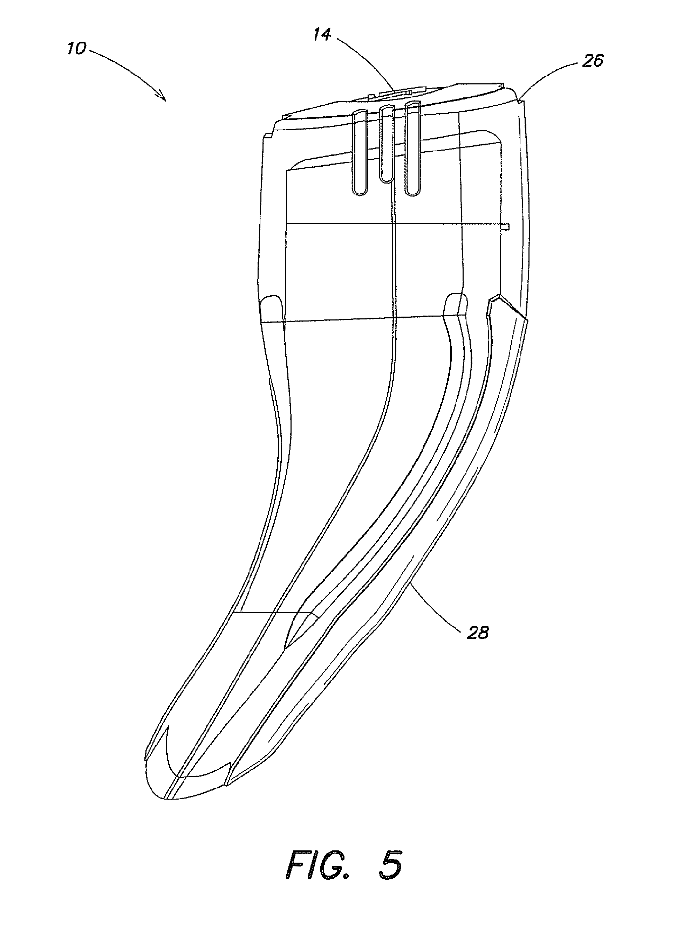

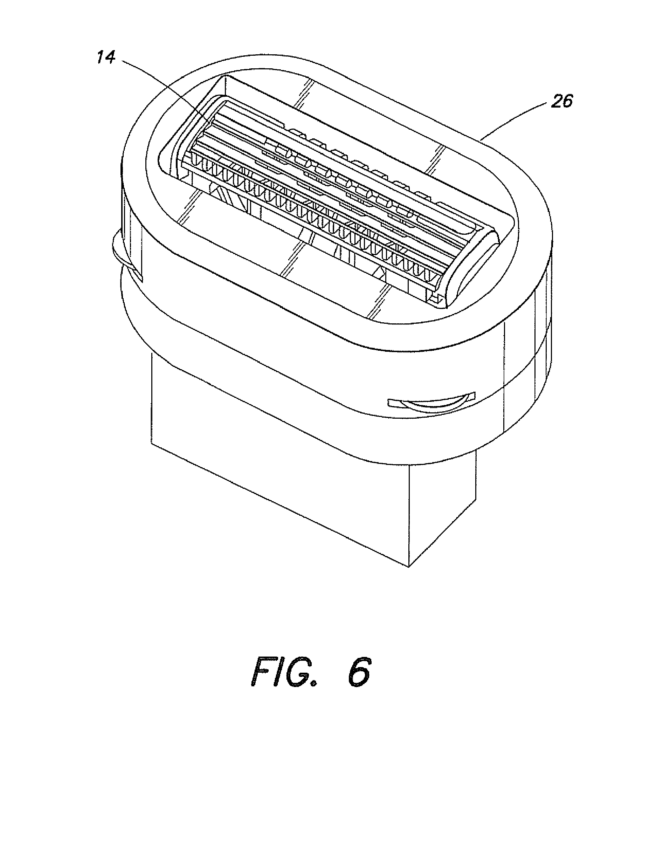

Referring to FIGS. 1-6, a razor assembly 10 includes a handle 12 and a cartridge 14. The handle 12 can be rigidly or pivotally attached to the cartridge 14. The cartridge 14 can be a disposable cartridge for use with a reusable handle, or the handle 12 and cartridge 14 can be combined into a unitary disposable razor assembly. The cartridge 14 includes a frame having a seat 16 and a cap 18, and one or more razor blades 20 disposed between the seat 16 and the cap 18. The razor blades 20 each have a lengthwise-extending cutting edge. Depending upon the application, the cartridge 14 may also include a guard 22 attached to the frame. In the embodiment shown in FIG. 2, the cartridge includes a strip 24 of shaving aid material (sometimes referred to as a "comfort strip") disposed on or in a contact surface of the cap 18. In the embodiment shown in FIGS. 3 and 4, a strip 24 of shaving aid material is disposed within a channel 25 disposed in the cartridge 14 that extends around the periphery of the razor blades 20. In these type embodiments, the shaving aid strip 24 can be attached to the cap 18 by any known means (e.g., pins, mechanical fasteners, adhesives, etc.). FIGS. 5 and 6 illustrate an alternative embodiment of a razor assembly 10 that includes a razor cartridge 14, a shaving aid body 26, a handle 28, and a linkage (not shown). The razor cartridge 14 is connected to the linkage, which linkage provides means for relative motion between the razor cartridge 14 and the shaving aid body 26. The shaving aid body 26 is a single oval having a center aperture in which the razor cartridge 14 is disposed; i.e., the shaving aid body 26 surrounds the razor cartridge 14. This embodiment of a razor assembly 10 and others are provided in U.S. Patent No. 7,266,895, which is hereby incorporated in its entirety The razor assembly embodiments shown in FIGS. 1-6, represent examples of how the present invention shaving aid materials may be utilized within a razor assembly The present invention shaving aid materials are not limited to these applications, however.

The shaving aid material is a composite that includes a water-soluble lubricious shaving aid in combination with a water-insoluble erodable medium. In some embodiments, the shaving aid material further includes a water-soluble thermoplastic polymer, and may also include a plasticizer. Optional additional ingredients include emulsifiers, surfactant, skin conditioners, fragrances, depilatory agents, cleaning agents, medicinal agents, etc., as will be described below.

An acceptable water-soluble shaving aid is polyethylene oxide ("PEO"). PEOs are commercially available, for example, from The Dow Chemical Company under the trademark Polyox® in a variety of different molecular weights including POLYOX WSR coagulant, POLYOX WSR N-750, etc. Those skilled in the art will appreciate that a PEO useful as a shaving aid may in fact be a mixture of different types of PEO (e.g., high molecular weight PEO in combination with low molecular weight PEO). In addition to PEO, or in some instances in place of PEO, the water-soluble shaving aid may include one or more of the following constituents: (A) a lubricating agent for reducing the frictional forces between the razor and the skin, e.g., a micro-encapsulated silicone oil; (B) an agent that reduces the drag between the razor parts and the shaver's face, e.g., a natural polysaccharide derived from plant materials such as guar gum; (C) an agent which modifies the chemical structure of the hair to allow the razor blade to pass through the whiskers very easily, e.g., a depilatory agent; (D) a cleaning agent which allows the whiskers and skin debris to be washed more easily from the razor parts during shaving, e.g., a silicone polyethylene oxide block copolymer and detergent such as sodium lauryl sulphate; (E) a medicinal agent for killing bacteria, or repairing skin damage and abrasions; (F) a cosmetic agent for softening, smoothing, conditioning or improving the skin; (G) a blood coagulant for the suppression of bleeding that may occur from nicks and cuts; and (H) an astringent for constricting blood vessels thereby stemming the flow of bodily fluids such as lymph, which may exude from skin which has been irritated during shaving. The water-soluble shaving aid may include constituents that by themselves are partially or completely water-insoluble, but in combination with other shaving aid constituents are water-soluble to an acceptable degree.

An acceptable erodable medium is one that may be described as being malleable at normal ambient temperatures, has a melting point above approximately 45°C (113°F), a relatively low viscosity when melted (e.g., below about 1000 centipoise; in contrast to a polymeric material that has a high viscosity once its temperature is raised above its glass transition temperature), is hydrophobic or water-insoluble, and has a molecular weight below about 25,000. In preferred embodiments, the erodable medium comprises an amphipathic material; i.e., a material comprised of molecules having a hydrophobic end and a hydrophilic end. Amphipathic materials have a "polar" nature that improves their compatibility with a "polar" material like PEO, and allows the materials to be more intimately mixed together; i.e., the nature of the amphipathic material is such that the PEO is at least partially miscible with the amphipathic material and thereby at least partially soluble with the erodable medium, which lowers the melt viscosity of the PEO and thereby creates a solution of PEO in "wax-like" material This is in contrast to conventional shaving aid materials that include composites formed from a mixture of water-soluble shaving aid (e.g., PEO) and water-insoluble matrix material (e.g., polystyrene, polypropylene, etc.). In such prior art shaving aid materials, the solubility parameters of PEO and the water-insoluble polymer make them substantially incompatible with one another. The water-insoluble polymer does not bond with the PEO in the conventional shaving aid material, but rather remains independent and acts as a matrix to support the PLO.

The compatibility of the water-soluble material and the water-insoluble erodable medium within the present shaving aid material favorably influences the longevity of the shaving aid material. When a conventional shaving aid material is exposed to water, the water-soluble PEO leaches out of the water-insoluble polymer matrix, and the water-insoluble polymer matrix is left behind. In fact, the water soluble shaving aid material(s) usually leaches out from the matrix of the conventional shaving aid faster than desired. The remaining polymer matrix may erode on the user's skin during shaving, but it will not perform acceptably as a shaving aid material. In contrast, the intimately mixed water-soluble shaving aid and water-insoluble erodable medium of the present invention (which water-soluble shaving aid is at least partially miscible and therefore at least partially in solution with the water-insoluble erodable medium) are applied to the user's skin surface in combination. Some of the water-soluble shaving aid that is not in solution with the water-insoluble erodable medium (i.e., "free" water-soluble shaving aid) may be applied to the user's skin independent of the erodable medium. The shaving aid that is in solution, however, erodes in combination with water-insoluble erodable material. As a result: 1) the erosion of the water-insoluble erodable material is facilitated and the combination of the two provides a significantly better quality shaving aid material than, for example, the polymeric matrix that remains when using conventional shaving aid materials; and 2) the longevity of the present invention shaving aid material is substantially better than that of conventional shaving aid materials. The erosion mechanism of the present invention shaving aid material is also favorable relative to prior art shaving aid materials that utilize a water-soluble polymeric matrix in combination with a water-soluble shaving aid such as PEO.

The amphipathic materials include fatty alcohols and fatty acids. Fatty alcohols are aliphatic alcohols derived from natural fats and oils, originating in plants, but also synthesized in animals and algae. Smaller molecule fatty alcohols are used in cosmetics and food, and as industrial solvents. Due to their amphipathic nature, fatty alcohols behave as nonionic surfactants. They find use as emulsifiers, emollients and thickeners in the cosmetics and food industries. Fatty alcohols are a common component of waxes, mostly as esters with fatty acids but also as alcohols themselves. Fatty acids may be described as a group of chemical compounds characterized by a chain made up of carbon and hydrogen atoms and having a carboxylic acid (COOH) group on one end of the molecule They differ from each other in the number of carbon atoms and the number and location of double bonds in the chain. When they exist unattached to the other compounds, they are called free fatty acids.

Amphipathic materials that can act as a surfactant typically have both lyophilic and lyophobic groups (properties) in the same molecule. These materials may also be referred to as being amphiphilic.

Examples of acceptable water-insoluble erodable mediums include stearic acid, palmitic acid, 12-hydroxystearic acid, stearyl alcohol (also referred to as octadecanol), cetyl alcohol (also referred to as hexadecanol), behenyl alcohol, cetearyl alcohol, arachidyl alcohol, C₂₀₋₄₀ and C₃₀₋₅₀ linear alcohols from New Phase Technologies under the trade name Performacol, paraffin wax alone or in combination with microcrystalline waxes; natural waxes including beeswax, carnauba wax, candelilla, and soy wax; silicone waxes such as C₃₀₋₄₅ alkyl methicone such as Dow Corning AMS-C30, Dow Corning 2503 Cosmetic Wax, Dow Corning Silky Wax; specialty waxes such as Emulium Kappa from Gatefosse; wax esters such as Synchrowaxes from Croda; hydrogenated castor oils such as Castorwax and esters such as gylceryl mono-hydroxystearate (e.g., NatureChem GMHS, from CasChem); and polyethyle waxes such as Polywax 400, Polywax 850, and Polywax 1000 offered by Baker Petrolite Polymer.

In some embodiments, the present invention shaving aid material further includes a water-soluble thermoplastic polymer. The main function of the water soluble thermoplastic polymer is to enhance the tensile strength of the material. Secondary functions are to increase the erodability of the material. Examples of useful polymers are Hydroxypropyl cellulose (e.g., Klucel from Aqualon), Polyvinylpyrrolidone (e.g., PVP from ISP) and Polyvinylpyrrolidone/Vinyl Acetate copolymers (e.g., Plasdone from ISP) and Poly (2-ethyl-2-oxazoline) (e.g., Aquazol from Polymer Chemistry Innovations, Inc.); hydroxypropyl starch; Grain Processing Zeina B860.

In some embodiments, the present invention shaving aid further includes one or more plasticizers as processing aids to improve the flexibility of the material. Examples are isostearic acid, Triacetin, and liquid fatty alcohols such as oleyl alcohol, isostearyl alcohol.

As stated above, the configuration of the shaving aid manner, its place of application to the razor cartridge, the manner of attachment and/or other means and method of incorporation may vary widely to fit particular design requirements.

The following shaving aid material formulations represent examples of the present invention shaving aid material, and the present invention is not limited to these examples. The following groups can be generally described as follows. Group 1 examples include at least a fatty alcohol, thermoplastic polymer, PEO and, optionally, an emulsifier. Group 1 includes examples A, B, C, D, E and K. Group 2 examples include at least a fatty alcohol, an emulsifier, and PEO. Group 2 includes examples F, G, H, and L. Group 3 examples include at least a fatty alcohol (or fatty acid) and PEO. Group 3 includes examples I and J. Group 4 examples include at least a fatty alcohol (or fatty acid), PEO and an anionic surfactant. Group 4 includes examples M, N, and O. Group 5 examples include at least a fatty alcohol, a cationic surfactant, and PEO. Group 5 includes example P.

### EXAMPLE A:

In Example A, the shaving aid material was formed from an erodable medium (Stearyl) alcohol, Glyceryl Hydroxystearate), water-soluble shaving aid (Polyox Coagulant, Polyox N-10), Isostearic acid, and Hydroxypropyl cellulose in the percentages provided below in Table 1.

**TABLE 1:**

| **Shaving Aid Material Example A Ingredients** | **Function** | **Ingredient %** |
|---|---|---|
| Stearyl alcohol | Erodable medium constituent; i.e., "waxy medium" | 35 |
| Glyceryl hydroxystearate | Erodable medium constituent; i.e., "waxy medium" | 7.5 |
| Isostearic acid | Plasticizer | 12.5 |
| Hydroxypropyl cellulose | Improves tensile strength; slows dissolution in hot water | 10 |
| Polyox coagulant | Water-soluble shaving aid | 27.5 |
| Polyox N-10 | Water-soluble shaving aid | 7.5 |

The materials were processed in a Sigma blade mixer. The Stearyl alcohol, Glyceryl hydroxystearate, and Isostearic acid were heated to about 225 °F until melted. The Hydroxypropyl cellulose was added and mixed until dissolved, and the Polyox materials were subsequently stirred into the mixture. The materials was subsequently poured onto release paper and pressed. The material was subsequently tested for performance.

### EXAMPLES B-D:

In Examples B-D, the shaving aid material was formed from an erodable medium (Stearyl alcohol), water-soluble shaving aid (Polyox coagulant, Polyox N-10), PVP/VA copolymer, and Hydroxypropyl cellulose in the percentages provided below in Table 2.

**TABLE 2**

| **Shaving Aid Material Examples B-D Ingredients** | **B** **Ingredient %** | **C** **Ingredient %** | **D** **Ingredient %** |
|---|---|---|---|
| Stearyl alcohol, | 60 | 65 | 65 |
| PVP/VA copolymer | 20 | 25 | 25 |
| Hydroxypropyl cellulose | 10 | 5 | -- |
| Polyox WSR N-10 | -- | -- | 5 |
| Polyox coagulant | 10 | 5 | 5 |

The shaving aid materials in Example B-D were processed in similar manner to that described above for Example A.

### EXAMPLE E:

In Example E, the shaving aid material was formed from an erodable medium (Stearyl alcohol), water-soluble shaving aid (Polyox coagulant), PVP/VA copolymer, Isostearic acid, and Glycerol triacetate in the percentages provided below in Table 3.

**TABLE 3**

| **Shaving Aid Material Example E Ingredients** | **Function** | **Ingredient %** |
|---|---|---|
| Stearyl alcohol | Erodable medium | 40 |
| Glyceryl hydroxystearate | Erodable medium | 10 |
| Isostearic acid | Plasticizer | 8 |
| Glycerol triacetate | Plasticizer | 2 |
| PVP/VA copolymer | Increases tensile strength | 20 |
| Polyox coagulant | Water-soluble shaving aid | 20 |

The materials for the shaving aid material in Example E were processed in a double arm mixer. The Stearyl alcohol, Glyceryl hydroxystearate, Isostearic acid, and Glycerol triacetate were heated to about 185 °F until melted, and the PVP/VA copolymer was added. The temperature of the mixture was increased to approximately 285°F until the PVP/VA copolymer was completely dissolved, and the Polyox was stirred into the mixture The material was subsequently poured onto release paper and pressed. The material was then tested for performance.

### EXAMPLES F-H:

In Examples F-H, the shaving aid material was formed from an erodable medium (Stearyl alcohol), water-soluble shaving aid (Polyox coagulant), Polyderm PPI-CO-200, Triacetin, and Macadamia nut oil in the percentages provided below in Table 4.

**TABLE 4:**

| Shaving Aid Material | **F** | **G** | **H** |
|---|---|---|---|
| Examples F-H Ingredients | **Ingredient %** | **Ingredient %** | **Ingredient %** |
| Stearyl alcohol | 20% | 40% | 20% |
| Polyderm PPI-CO-200 | 57% | 40% | 57% |
| Triacetin | 8% | 5% | -- |
| Macadamia nut oil | -- | -- | 8% |
| Polyox coagulant (ground fine) | 15% | 15% | 15% |

The materials for the shaving aid material in Examples F-H were processed in a double arm mixer. The Stearyl alcohol, polyderm, and triacetin were heated to about 165°F The Polyox and macadamia nut oil were subsequently stirred into the mixture, and the temperature was raised to 185°F. As the mixing continued, the material thickened and much of the Polyox dissolved.

### EXAMPLES I and J:

In Example I, a shaving aid material comprising 50% stearyl alcohol and 50% PEO was mixed. In Example J, a shaving aid material comprising 50% stearic acid, and 50% PEO coagulant was mixed. In both examples, the shaving aid materials were injection molded at temperatures at a maximum temperature of 80°C and a pressure of 36,000.

### EXAMPLE K:

In Example K, the shaving aid material was formed from an erodable medium (Stearyl alcohol, Glyceryl hydroxystearate), water-soluble shaving aid (Polyox coagulant), Isostearic acid, Glycerol triacetate, and PVP/VA copolymer in the percentages provided below in Table 5.

**TABLE 5:**

| **Shaving Aid Material Example E Ingredients** | **Function** | **Ingredient %** |
|---|---|---|
| Stearyl alcohol | Erodable medium | 42.5 |
| Glyceryl hydroxystearate | Erodable medium | 10 |
| Orange colorant | Colorant | 0.25 |
| Hydroxypropyl cellulose | Increase tensile strength | 10 |
| PVP/VA copolymer | Increase tensile strength | 20 |
| Polyox coagulant | Water-soluble shaving aid | 17.5 |

In Example K, the shaving aid materials were mixed and injection molded at temperatures in the range of about 140°F - 160°F.

In Example L, a shaving aid material was prepared comprising 25% stearyl alcohol, 61% C₂₀₋₄₀ pareth-40 (Performathox 480 from New Phase Technology) and 15% Polyox coagulant was prepared by melting the mixture to about 90°C and pouring into a mold.

In Examples M and N, the following ingredients were heated, mixed and injection molded into shaving strips:

| **Ingredient** | **function** | **wt% (example M)** | **wt% (Example N)** |
|---|---|---|---|
| Stearic acid, Acme Hardesty 90% | wax | 30 | 30 |
| sodium cocoyl isethionate (Hostapon SCI-85P from Clariant) | surfactant | 40 | |
| sodium N-acyl-L-glutamate (Amisoft GS 11-P from Ajinomoto) | surfactant | | 40 |
| PEG-115M (PEO coagulant from Dow) | slippery polymer | 30 | 15 |
| PEG-7M (WSRN-750 from Dow) | slippery polymer | | 15 |

Example O was was extruded which contained 85% of a commercial mixture of disodium lauryl sulfosuccinate, sodium cocoyl isethionate, cetearyl alcohol, triticum vulgare (wheat) starch, glyceryl stearate, paraffin, titanium dioxide (Zetasap ST 5251 from Zschimmer & Schwarz) and 15% PEO coagulant.

Although this invention has been shown and described with respect to the detailed embodiments thereof, it will be understood by those skilled in the art that various changes in form and detail thereof may be made without departing from the spirit and scope of the invention.

According to further embodiments, the present invention may comprise one or more of the following features:
A shaving aid material, comprising:
   a water-soluble shaving aid; and
   a water-insoluble erodable medium that has a melting point above about 45°C, and has a molecular weight below about 25,000; and
   wherein the water-soluble shaving aid is at least partially soluble or miscible with the water-insoluble erodable medium.

A shaving aid material, wherein the water-soluble shaving aid includes PEO.

A shaving aid material, wherein the shaving aid material includes a water-soluble thermoplastic polymer.

A shaving aid material, wherein the water-soluble thermoplastic polymer includes at least one of Hydroxypropyl cellulose, Polyvinylpyrrolidone, Polyvinylpyrrolidone/Vinyl Acetate copolymers, Poly (2-ethyl-2-oxazoline), and hydroxypropyl starch.

A shaving aid material, wherein the shaving aid material includes a plasticizer.

A shaving aid material wherein the plasticizer includes at least one of isostearic acid, Triacetin, oleyl alcohol, and isostearyl alcohol.

A shaving aid material, wherein the shaving aid material includes an emulsifier.

A shaving aid material, wherein the water-insoluble erodable medium is malleable at normal ambient temperatures.

A shaving aid material, wherein the water-insoluble erodable medium has a viscosity, when melted, that is below about 1000 centipoise,

A shaving aid material, wherein the water-insoluble erodable medium comprises an amphipathic material.

A shaving aid material, wherein the amphipathic material includes a fatty alcohol.

A shaving aid material, wherein the amphipathic material includes a fatty acid.

A shaving aid material, wherein the water-insoluble erodable mediums include at least one of stearic acid, palmitic acid, 12-hydroxystearic acid, stearyl alcohol, cetyl alcohol, behenyl alcohol, cetearyl alcohol, arachidyl alcohol, C₂₀₋₄₀ and C₃₀₋₅₀ linear alcohols, paraffin wax, beeswax, carnauba wax, candelilla, soy wax, silicone wax, wax esters, hydrogenated castor oil, and polyethyle wax.

A cartridge, comprising:
frame;
at least on blade positioned in the frame; and
a shaving aid material, the shaving aid material comprising:
   a water-soluble shaving aid; and
   a water-insoluble erodable medium that has a melting point above about 45°C, and has a molecular weight below about 25,000; and
   wherein the water-soluble shaving aid is at least partially soluble or miscible with the water-insoluble erodable medium.

A cartridge, wherein the shaving aid material is positioned aft of at least one of the blade(s).

A cartridge, wherein the shaving aid material is positioned forward of at least one the blade(s).

A cartridge, wherein the shaving aid material includes an aperture such that the shaving aid material substantially surrounds the blade(s).

A cartridge, wherein the shaving aid material is affixed to the frame.

A cartridge, wherein the shaving aid material is movable relative to the frame.

## Claims

1. A method of making a shaving aid material, the steps for making the shaving aid material comprising:
- providing an amount of at least one of a fatty alcohol or a fatty acid;
- providing an amount of PEO;
- heating the PEO and either or both of the fatty alcohol and fatty acid to a temperature less than about 115°C;
- mixing the PEO and either or both of the fatty alcohol and fatty acid to create a mixture; and
- forming mixture into a shaving strip.

2. The method of claim 1, wherein an anionic surfactant is also heated and mixed into the mixture prior to forming.

3. The method of claim 2, wherein the shaving strip is formed through an extrusion process.

4. The method of claim 3, wherein the temperature of the mixture is less than 95°C during the extrusion process.

5. The method of claim 4, wherein the temperature of the mixture is less than 52°C during the extrusion process

6. The method of claim 1, wherein a cationic surfactant is also heated and mixed into the mixture prior to forming the shaving strip.

7. The method of claim 1, wherein an emulsifier is also heated and mixed into the mixture prior to forming the shaving strip.

8. The method of claim 7, wherein the forming process includes pouring the mixture.

9. The method of claim 8, wherein the temperature of the mixture is less than 90°C during pouring,

10. The method of claim 9, wherein the temperature of the mixture is less than 85°C during pouring.

11. The method of claim 1, wherein a thermoplastic polymer is heated and mixed into the mixture prior to forming.

12. The method of claim 11, wherein an emulsifier is also heated and mixed into the mixture prior to forming.

13. The method of claim 12, wherein the forming process includes pouring the mixture.

14. The method of claim 13, wherein the temperature of the mixture is less than 110°C during pouring.

15. The method of claim 11, wherein the forming process includes injection molding the mixture into a mold.

16. The method of claim 15, wherein the temperature of the mixture is less than 75°C during injection molding, and the pressure is less than 10000psi.

17. The method of claim 1, wherein the forming process includes injection molding the mixture into a mold.

18. The method of claim 17, wherein the temperature of the mixture is less than 80°C during injection molding, and the pressure is less than 40000psi.
